# EUROPÄISCHE PATENTSCHRIFT

(11) **EP 0 381 624 B1**
(45) Veröffentlichungstag und Bekanntmachung des Hinweises auf die Patenterteilung: **12.05.1993**
(21) Anmeldenummer: 90810058.9
(22) Anmeldetag: 24.01.1990
(51) Int. Cl.: C07C 323/22, C07C 319/14, C07C 47/14

(54) **Verfahren zur Herstellung von 1-Alkylthio- und 1-Benzylthio-1-formylcyclopropanen**
Process for the production of 1-alkyl thio- and of 1-benzyl thio-1-formyl-cyclopropanes
Procédé pour la préparation d'alkylthio-1 et de benzylthio-1-formyl-1 cyclopropanes

(30) Priorität: 02.02.1989 CH 366/89
(43) Veröffentlichungstag der Anmeldung: 08.08.1990
(73) Patentinhaber: CIBA-GEIGY AG, 4002 Basel (CH)
(72) Erfinder: Tobler, Hans, Dr., CH-4123 Allschwil (CH)

(56) Entgegenhaltungen:
- CH-A- 619 441
- DE-A- 2 120 908
- DE-A- 2 403 236

## Beschreibung

Die vorliegende Erfindung betrifft ein neues Verfahren zur Herstellung von 1-Alkylthio- und 1-Benzylthio-1-formylcyclopropanen der Formel I 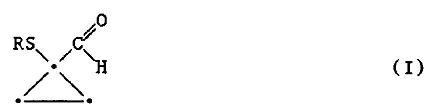
worin R für C₁-C₄-Alkyl oder Benzyl steht.

Unter Alkyl ist geradkettiges oder verzweigtes Alkyl zu verstehen. Geeignete C₁-C₄-Alkylreste sind beispielsweise Methyl, Ethyl, n-Propyl, i-Propyl, n-Butyl oder die isomeren Butyl.

Die 1-Alkylthio- und 1-Benzylthio-1-formylcyclopropane der Formel I sind wertvolle Ausgangsverbindungen für die Herstellung von herbizid wirksamen Acyl-cyclohexandionen wie sie beispielsweise in der Europäischen Patentanmeldung EP-A-243 313 beschrieben sind.

Aus der Deutschen Offenlegungsschrift DOS 2 120 908 ist bekannt, 1-Methylthio-1-formylcyclopropan in der Weise herzustellen, dass man
a) Methylthioacetonitril in Gegenwart von Natriumamid mit 1,2-Dibromethan zum 1-Cyano-1-methylthiocyclopropan der Formel V 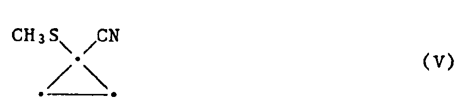 umsetzt,
b) dieses mit konzentrierter Salzsäure in die entsprechende Cyclopropancarbonsäure der Formel VI 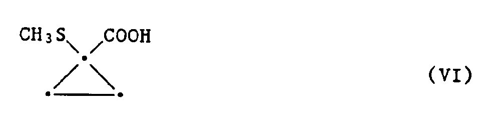 überführt,
c) die Carbonsäure der Formel VI mit Thionylchlorid zum Säurechlorid der Formel VII 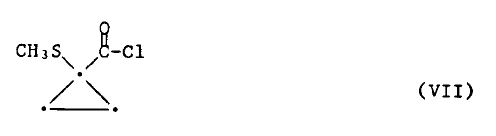 umsetzt,
d) das so gebildete Säurechlorid mit Ethylenimin und Triethylamin zum Amid der Formel VIII 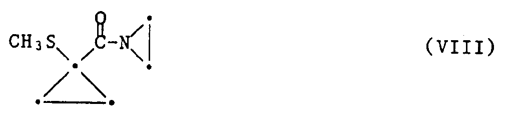 reagieren lässt um schliesslich
e) durch Umsetzung mit Lithiumaluminiumhydrid das Endprodukt 1-Formyl-1-methylthiocyclopropan zu erhalten.

Die mit diesem bekannten Verfahren erzielbaren Ausbeuten sind sehr gering, so wird beispielsweise beim Verfahrensschritt a) die Verbindung der Formel V nur in einer Ausbeute von 16,8 % d.Th. erhalten.

Bezogen auf das Ausgangsprodukt Methylthioacetonitril beträgt die Ausbeute an Endprodukt nach diesem 5-stufigen Verfahren nur ca. 4 %.

Neben der für grosstechnisch anwendbare Verfahren unbefriedigenden Ausbeute ist die Durchführung dieses bekannten Verfahrens durch die grosse Anzahl der Synthese- und Aufarbeitungsschritte sehr zeitaufwendig und kostenintensiv.

Es wurde nun ein neues Verfahren gefunden, welches die Herstellung von 1-Alkylthio- und 1-Benzylthio-1-formylcyclopropanen auf besonders einfache Weise und in hohen Ausbeuten und guter Reinheit ermöglicht.

Das erfindungsgemässe Verfahren zur Herstellung von Verbindungen der Formel I, 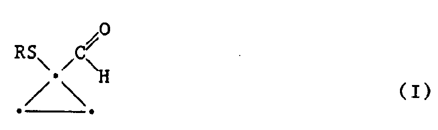
worin
R C₁-C₄-Alkyl oder Benzyl bedeutet, ist dadurch gekennzeichnet, dass man ein Butanal der Formel II, 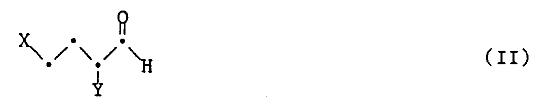
worin X und Y unabhängig voneinander für Chlor, Brom und Jod stehen, in einem inerten Lösungsmittel mit einer Verbindung der Formel III

R-S-Me (III)

worin R die oben angegebene Bedeutung hat und Me für ein Alkalimetallkation steht, umsetzt, und die auf diese Weise erhaltene Verbindung der Formel IV 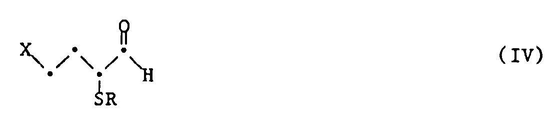
worin X und R die oben angegebene Bedeutung haben, in einem inerten Lösungsmittel in Gegenwart einer Base in eine Verbindung der Formel I überführt.

Für das erfindungsgemässe Verfahren geeignete Lösungsmittel sind beispielsweise Verbindungen oder Gemische aus der Gruppe der offenkettigen und zyklischen Ether wie Dioxan, Tetrahydrofuran, Diethylether, Diisopropylether, Dimethoxymethan und 1,2-Dimethoxyethan oder aus der Gruppe der Alkohole wie Methanol, Ethanol, Propanol, Isopropanol und Butanol oder Wasser. Als besonders gut geeignete Lösungsmittel haben sich Methanol, Tetrahydrofuran und Wasser erwiesen.

Wie oben bereits beschrieben, ist Me ein Alkalimetall; insbesondere aber Lithium, Natrium und Kalium.

Beispiele für geeignete Basen sind Oxide, Hydroxide, Carbonate, Carbonsäuresalze oder Alkoholate eines Alkali- oder Erdalkalimetalls. Als besonders gut geeignet haben sich Natriummethylat, Natriumhydroxid, Kaliumhydroxid, Natriumcarbonat oder Kaliumcarbonat erwiesen. Besonders bevorzugt ist Natriummethylat, Natriumhydroxid und Kaliumhydroxid.

Das erfindungsgemässe Verfahren kann in einem weiten Temperaturbereich durchgeführt werden. Als gut geeignet hat sich ein Temperaturbereich von -10°C bis +100°C erwiesen, bevorzugt ist ein Temperaturbereich von -10°C bis +50°C.

Die Thiolsalze der Formel III können sowohl als solche verwendet als auch in situ erzeugt werden. Beispielsweise kann Natriumthiomethylat durch Einleiten von Methylmercaptan in eine wässrige Natriumhydroxidlösung hergestellt werden.

Die Ausgangsverbindungen der Formel II sind neu und ebenfalls Gegenstand der Erfindung. Sie können analog zu literaturbekannten Verfahren aus den entsprechenden 4-Halogenbutanalen hergestellt werden. Beispiele für solche Synthesen finden sich in:
J. Org. Chem. (1974), 39, 1785;
Chem. Ber. (1985), 118, 4288;
Synthesis (1978), 140;
J. Org. Chem. (1983), 48, 3493;
Synthesis (1986), 678;

Die Zwischenprodukte der Formel IV sind neue Verbindungen und bilden einen Gegenstand dieser Erfindung.

Das erfindungsgemässe Verfahren kann sowohl mit als auch ohne Isolierung der Zwischenprodukte der Formel IV durchgeführt werden.

In einer bevorzugten Variante des erfindungsgemässen Verfahrens setzt man die Verbindung der Formel II mit der Verbindung der Formel III ohne Isolierung des Zwischenproduktes der Formel IV in einem inerten Lösungsmittel in Gegenwart einer Base zur Verbindung der Formel I um.

Für die Synthese der Verbindungen der Formel I besonders gut geeignete Ausgangsstoffe der Formel II sind jene, bei denen X für Chlor und Y für Chlor oder Brom steht. Ganz besonders gut geeignet sind Verbindungen der Formel II, worin X und Y für Chlor stehen.

In einer ganz besonders bevorzugten Variante des erfindungsgemässen Verfahrens setzt man eine Verbindung der Formel II, worin X für Chlor und Y für Chlor oder Brom stehen, direkt in einem Eintopfverfahren bei einer Temperatur von -10°C bis +50°C in Gegenwart von wässrigem Natriumhydroxid mit Natriumthiomethylat um.

Mit dem erfindungsgemässen Verfahren können die Verbindungen der Formel I auf besonders einfache Weise in hoher Reinheit und guter Ausbeute hergestellt werden. Durch die nur ein- bzw. - falls eine Isolierung des Zwischenproduktes der Formel IV gewünscht wird - zweistufige Verfahrensführung entfallen die zahlreichen zeitaufwendigen und gewöhnlich zu Ausbeuteverlusten führenden Aufarbeitungsschritte des bekannten Verfahrens.

Die nachfolgenden Beispiele dienen der Erläuterung der Erfindung. Sie sollen sie in keiner Weise beschränken.

### Herstellungsbeispiele:

### Beispiel 1: Herstellung von 2-Brom-4-chlorbutanal (Verbindung Nr. 1.1)

Zu einer Suspension aus 120 g 4-Chlorbutanal und 130 g 5,5-Dibrombarbitursäure in 600 ml Diethylether lässt man unter intensivem Rühren 12 ml einer 33%igen Bromwasserstofflösung in Essigsäure zutropfen, wobei nach Einsetzen der Reaktion die Temperatur auf +35°C steigt. Nach 3 bis 4 Stunden wird die auf Raumtemperatur abgekühlte Reaktionsmischung filtriert. Anschliessend wird das Filtrat nacheinander mit verdünnter Natriumhydrogencarbonatlösung und mit gesättigter Natriumchloridlösung neutral gewaschen und mit Natriumsulfat getrocknet. Nach Abdampfen des Lösungsmittels bei +40°C erhält man 133 g 2-Brom-4-chlorbutanal in einer Ausbeute von 70 % d.Th. und mit einer Reinheit von 93 %. Der Siedepunkt nach destillativer Reinigung bei 1900 Pa Druck beträgt +74 bis +75°C.

### Beispiel 2: Herstellung von 2,4-Dichlorbutanal (Verbindung Nr. 1.2)

Zu einer Lösung von 237 g 4-Chlorbutanal mit einem Gehalt von 90 % in 80 ml Dichlormethan lässt man innerhalb von 75 Minuten unter Eiskühlung 270 g Sulfurylchlorid zutropfen. Während dieser Massnahme beträgt die Temperatur der Reaktionsmischung +10 bis +20°C. Die Reaktionsmischung wird anschliessend für 30 Minuten bei einer Temperatur von +5 bis +10°C gehalten und dann zwei Stunden lang unter Rückfluss bis zum Ende der Gasentwicklung erhitzt. Das Reaktionsprodukt erhält man schliesslich durch fraktionierte Destillation über eine 40 cm Füllkörperkolonne bei 6000 Pa in einer Menge von 221 g mit einem Siedepunkt von +90°C. Nach Reinigung durch Feindestillation bei 9000 Pa über eine 20 cm Füllkörperkolonne erhält man 194,1 g (54,7 % d.Th.) 2,4-Dichlorbutanal in einer Reinheit von 79,5 % und mit einem Siedepunkt von +96 bis +99°C.

### Beispiel 3: Herstellung von 4-Chlor-2-jodbutanal (Verbindung Nr. 1.3)

Eine Suspension von 59,1 g 4-Chlorbutanal mit einem Gehalt von 90 %, 68 g Quecksilber-II-chlorid und 127 g Jod in 500 ml Dichlormethan wird mit 1,5 ml 57%ige Jodwasserstoffsäure versetzt und 24 Stunden unter Rückfluss gerührt. Während dieser Massnahme entweicht aus der Reaktionsmischung langsam Chlorwasserstoff bei Bildung von rotem Quecksilber-II-jodid. Die Reaktionslösung wird filtriert und das Filtrat nach zweimaligem Waschen mit Natriumthiosulfatlösung und einmaligem Waschen mit einer Lösung aus Kaliumjodid und Natriumhydrogencarbonat mit Magnesiumsulfat getrocknet. Anschliessend wird das Filtrat eingedampft und über eine 30 cm Füllkörperkolonne fraktioniert destilliert. Man erhält 85,3 g 4-Chlor-2-jodbutanal in Form eines gelbrot gefärbten Oeles in einer Ausbeute von 61 % d.Th. und einem Gehalt von 84 %. Der Siedepunkt beträgt +46 bis +47°C bei 1,3 Pa.

### Beispiel 4: Herstellung von 2,4-Dibrombutanal (Verbindung Nr. 1.4)

Zu einer Suspension aus 70 g 4-Brombutanal mit einem Gehalt von 98,8 % und 65,5 g 5,5-Dibrombarbitursäure in 300 ml Diethylether lässt man unter intensivem Rühren 1,4 ml 33%ige Bromwasserstofflösung in Essigsäure zutropfen. Die Temperatur der Reaktionsmischung steigt innerhalb von 2 Stunden auf +31°C an. Nach weiteren 2 Stunden ist die Reaktionsmischung wieder auf Raumtemperatur abgekühlt. Man kühlt die Mischung auf 0°C und filtriert. Anschliessend wird das Filtrat mit gesättigter Natriumchloridlösung und etwas Natriumhydrogencarbonatlösung dreimal gewaschen. Nach Trocknen des Filtrates über Magnesiumsulfat und anschliessendem Eindampfen der Lösung erhält man 90 g eines blassgelben Oeles mit einem Produktgehalt von 91,1 %.

Nach destillativer Aufarbeitung des Rohproduktes über eine Vigreux-Kolonne bei 1,3 Pa erhält man 39,7 g 2,4-Dibrombutanal (34 % d.Th.) mit einem Siedepunkt von +42 bis +45°C/1,3 Pa und einem Gehalt von 92 % in Form eines farblosen, leicht beweglichen Oeles.

Entsprechend der vorstehend beschriebenen Arbeitsweise werden auch die folgenden Verbindungen der Formel II erhalten:

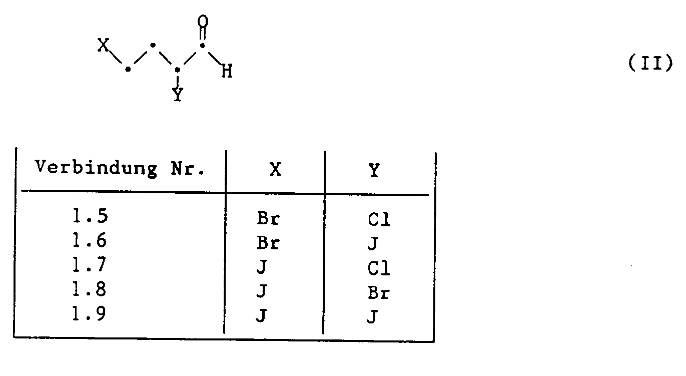

### Beispiel 5: Herstellung von 4-Chlor-2-methylthiobutanal (Verbindung Nr. 3.1)

Zu 54,8 g frisch destilliertem 2,4-Dichlorbutanal (96 %) in 100 ml Tetrahydrofuran gibt man bei einer Temperatur von -5°C innerhalb von 20 Minuten eine Lösung von 26,2 g Natriumthiomethylat in 170 ml Methanol tropfenweise hinzu. Nach Halten über 18 Stunden bei Raumtemperatur wird die Reaktionsmischung mit Diethylether verdünnt und anschliessend mit Wasser extrahiert. Anschliessend wird die organische Phase zweimal mit gesättigter Natriumchloridlösung und einmal mit Natriumhydrogencarbonatlösung gewaschen. Nach Trocknen über Magnesiumsulfat und Einengen über eine Kolonne bei Atmosphärendruck wird die Reaktionsmischung über eine 30 cm Füllkörperkolonne bei 2600 Pa destilliert. Man erhält 6,7 g 4-Chlor-2-methylthiobutanal (Verbindung Nr. 3.1) in Form eines farblosen Oeles mit einem Siedepunkt von +104°C bei 2600 Pa.

### Beispiel 6: Herstellung von 1-Formyl-1-methylthiocyclopropan (Verbindung Nr. 2.1)

Zu 6 g 4-Chlor-2-methylthiobutanal (Verbindung Nr. 3.1) in 2 ml Methanol gibt man bei +25°C 15 ml 3 N wässrige Natriumhydroxidlösung hinzu. Nach 35-minütigem Rühren wird die Reaktionsmischung zweimal mit Diethylether extrahiert und anschliessend mit gesättigter Natriumchloridlösung gewaschen. Die vereinigten organischen Phasen werden mit Magnesiumsulfat getrocknet. Nach Einengen der Lösung über eine Kolonne bei Atmosphärendruck wird der erhaltene Rückstand im Kugelrohr bei +150 bis +200°C bei Atmosphärendruck destilliert. Man erhält 2,4 g 1-Formyl-1-methylthiocyclopropan (Verbindung Nr. 2.1) in Form eines Oeles.

### Beispiel 7: Herstellung von 1-Formyl-1-methylthiocyclopropan (Verbindung Nr. 2.1)

Zu einer unter Eiskühlung hergestellten Lösung aus 58,6 g 2-Brom-4-chlorbutanal (frischdestilliert, Gehalt 95 %) in 50 ml Methanol lässt man unter Kühlung innerhalb von 15 Minuten 22,4 g 94%iges Natriumthiomethylat in 130 ml Methanol zutropfen. Dabei beträgt die Temperatur der Reaktionsmischung +20 bis +23°C. Nach 15 Minuten lässt man zu dem entstandenen Zwischenprodukt 4-Chlor-2-methylthiobutanal (Verbindung Nr. 3.1) innerhalb von 1 bis 2 Minuten unter Eiskühlung 54 g 30%ige methanolische Natriummethylatlösung zutropfen. Bei dieser Massnahme steigt die Temperatur der Reaktionsmischung auf +30°C. Nach Abklingen der Reaktion wird die Mischung mit Wasser versetzt und anschliessend dreimal mit Diethylether extrahiert. Die vereinigten organischen Phasen wäscht man mit gesättigter Natriumchloridlösung und trocknet danach über Magnesiumsulfat. Nach Einengen der Lösung bei Atmosphärendruck über eine Kolonne destilliert man den Rückstand fraktioniert über eine Füllkörperkolonne. Man erhält 23,8 g (67 % d.Th.) 1-Formyl-1-methylthiocyclopropan, welches einen Siedepunkt von +88 bis +91°C bei 9600 bis 10000 Pa und einen Gehalt von 98 % aufweist.

### Beispiel 8: Herstellung von 1-Formyl-1-methylthiocyclopropan (Verbindung Nr. 2.1)

Zu einer Lösung von 81 g 2,4-Dichlorbutanal mit einem Gehalt von 92 % in 300 ml Tetrahydrofuran gibt man unter Eiskühlung innerhalb von 30 Minuten portionsweise 38 g Natriumthiomethylat. Dabei entsteht das Zwischenprodukt 4-Chlor-2-methylthiobutanal (Verbindung Nr. 3.1). Die Reaktionsmischung hält man 24 Stunden bei Raumtemperatur und gibt dann bei 0°C innerhalb von 15 Minuten unter intensivem Kühlen 95,4 g 30%ige methanolische Natriummethylatlösung hinzu. Anschliessend lässt man die Reaktionsmischung auf Raumtemperatur erwärmen. Nach Extraktion durch Zugabe von 250 ml Wasser und 250 ml Diethylether trennt man die wässrige Phase ab. Diese wird ein zweites Mal mit 250 ml Diethylether extrahiert. Anschliessend wäscht man die vereinigten organischen Phasen zweimal mit je 250 ml gesättigter Natriumchloridlösung, trocknet über Magnesiumsulfat und engt die Lösung bei Atmosphärendruck über eine Kolonne ein. Nach fraktionierter Destillation des Rückstandes über eine 30 cm Füllkörperkolonne erhält man 30,5 g (47 % d.Th.) 1-Formyl-1-methylthiocyclopropan mit einem Gehalt von 97 % und einem Siedepunkt von +92°C bei 10200 bis 10400 Pa.

### Beispiel 9: Herstellung von 1-Formyl-1-methylthiocyclopropan (Verbindung Nr. 2.1)

720 g 30%ige methanolische Natriummethylatlösung (2 Aequ.) werden mit 100 ml Methanol verdünnt. In diese Lösung leitet man unter Eis/Kochsalz-Kühlung bei 0 bis +5°C 90 g Methylmercaptan ein wobei Natriumthiomethylat entsteht. Anschliessend lässt man zu der Reaktionslösung innerhalb von 20 Minuten 264 g frisch destilliertes 2,4-Dichlorbutanal zutropfen. Unter sofortigem Ausfallen von Natriumchlorid steigt die Temperatur der Reaktionsmischung auf +37°C. Nach 90 Minuten wird die Reaktionsmischung unter Zugabe von 1 l Wasser und 1 l Diethylether extrahiert. Anschliessend trennt man die organische Phase ab und extrahiert die wässrige Phase weitere viermal mit je 250 ml Diethylether. Nach Waschen der vereinigten organischen Phasen mit gesättigter Natriumchloridlösung und Trocknen über Magnesiumsulfat wird die Reaktionsmischung über eine Füllkörperkolonne bei Atmosphärendruck eingeengt. Nach fraktionierter Destillation des verbliebenen Rückstandes mit einer 40 cm Füllkörperkolonne erhält man 168 g (75 % d.Th.) 1-Formyl-1-methylthiocyclopropan mit einem Gehalt von 97 % und einem Siedepunkt von +92 bis +93°C bei 10400 Pa.

### Beispiel 10: Herstellung von 1-Formyl-1-methylthiocyclopropan (Verbindung Nr. 2.1)

Zu einer Lösung aus 107,5 g pulverisiertem Kaliumhydroxid (85 %) in 600 ml Methanol setzt man unter Rühren 120,3 g Natriumthiomethylat (Gehalt: 97 %) hinzu. Nach Kühlen der klaren, gelblichen Lösung auf +15°C lässt man unter Eiskühlung innerhalb von 25 Minuten 230 g 2,4-Dichlorbutanal mit einem Gehalt von 98 % gelöst in 200 ml Tetrahydrofuran zutropfen. Die Temperatur der Reaktionsmischung beträgt dabei +30 bis +40°C. Nach einer Stunde gibt man 1 l Wasser hinzu und extrahiert viermal mit je 300 ml Diethylether. Anschliessend werden die vereinigten organischen Phasen mit gesättigter Natriumchloridlösung gewaschen. Nach Trocknen über Magnesiumsulfat engt man die Lösung über eine Füllkörperkolonne bei Atmosphärendruck ein. Man erhält nach fraktionierter Destillation des Rückstandes bei reduziertem Druck über eine 40 cm Füllkörperkolonne 135,8 g 1-Formyl-1-methylthiocyclopropan (71,8 % d.Th.) mit einem Gehalt von 99 % und einem Siedepunkt von +89 bis +91°C bei 9600 Pa.

### Beispiel 11: Herstellung von 1-Formyl-1-methylthiocyclopropan (Verbindung Nr. 2.1)

In einer Lösung aus 116,3 g Natriumhydroxid (2 Aequ.) in 750 ml Wasser leitet man unter Eis/Natriumchloridkühlung 70 g Methylmercaptan (1 Aequ.) ein. Anschliessend lässt man unter Aufrechterhaltung der Kühlung bei intensivem Rühren innerhalb von 100 Minuten 188,6 g frisch destilliertes 2,4-Dichlorbutanal (Gehalt 97 %) zutropfen. Bei dieser Massnahme steigt die Temperatur der Reaktionsmischung von +2°C auf +8 bis +9°C an. Nach einer Stunde wird die Reaktionsmischung viermal mit je 250 ml Diethylether extrahiert. Die vereinigten organischen Phasen werden mit gesättigter Natriumchloridlösung neutral gewaschen und anschliessend über Magnesiumsulfat getrocknet. Nach Einengen der Lösung über eine Füllkörperkolonne bei Atmosphärendruck und anschliessendem fraktioniertem Destillieren über eine 40 cm Füllkörperkolonne bei reduziertem Druck erhält man 126 g 1-Formyl-1-methylthiocyclopropan (85 % d.Th.) mit einer Reinheit von 99 % und einem Siedepunkt von +91 bis +92°C bei 10000 Pa.

### Beispiel 12: Herstellung von 1-Formyl-1-tert.-butylthiocyclopropan (Verbindung Nr. 2.2)

Unter einer Stickstoffatmosphäre lässt man zu einer auf +5°C gekühlten Lösung von 80 g Natriumhydroxid in 750 ml Wasser unter Eiskühlung innerhalb von 10 Minuten 112 ml tert.-Butylmercaptan zutropfen. Anschliessend werden 50 ml Methanol zugegeben. Nach einer Stunde lässt man unter intensivem Rühren und Kühlen innerhalb von 15 Minuten 151 g 2-Brom-4-chlorbutanal (Gehalt 89,5 %) zutropfen, wobei die Temperatur der Reaktionsmischung von +5°C auf +20°C steigt. Nach 30 Minuten wird die Mischung viermal mit Diethylether extrahiert. Anschliessend werden die vereinigten organischen Phasen zweimal mit gesättigter Natriumchloridlösung gewaschen und über Magnesiumsulfat getrocknet. Der durch Eindampfen der Lösung am Rotationsverdampfer erhaltene Rückstand wird über eine 30 cm Füllkörperkolonne fraktioniert destilliert. Man erhält 89 g 1-Formyl-1-tert.-butylthiocyclopropan (Gehalt 89 %) mit einem Siedepunkt von +112 bis +114°C bei einem Druck von 8400 Pa.

Nach vorstehend beschriebenen Arbeitsweisen werden auch die folgenden Verbindungen der Formel I erhalten:

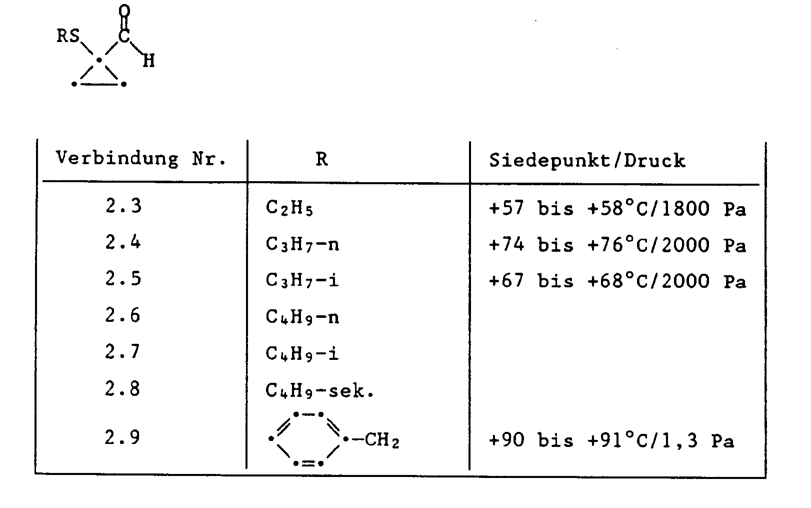

Nach den in den Beispielen 5, 7 und 8 beschriebenen Arbeitsweisen werden ebenfalls die folgenden Zwischenprodukte der Formel IV erhalten:

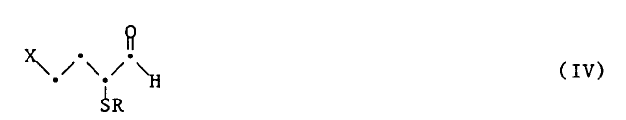

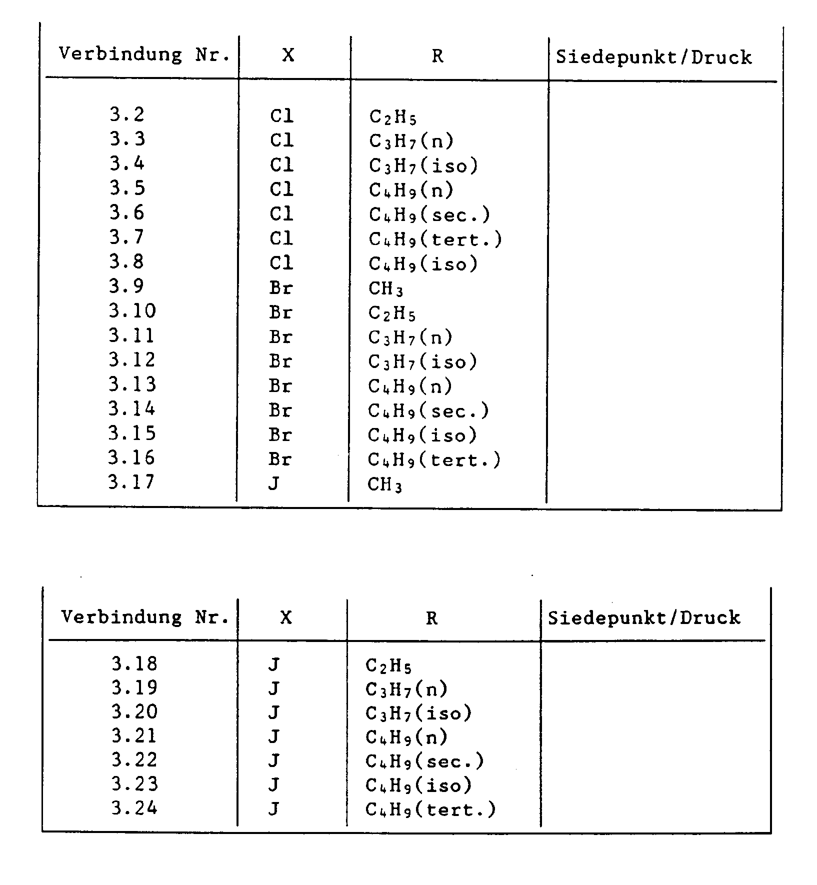

## Patentansprüche

1. Verfahren zur Herstellung von Verbindungen der Formel I, 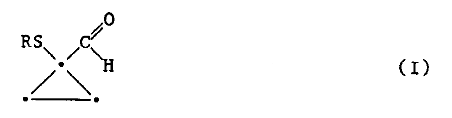 worin
R C₁-C₄-Alkyl oder Benzyl bedeutet, dadurch gekennzeichnet, dass man ein Butanal der Formel II, 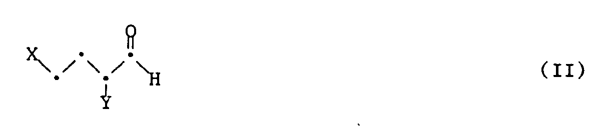 worin X und Y unabhängig voneinander für Chlor, Brom und Jod stehen, in einem inerten Lösungsmittel mit einer Verbindung der Formel III
R-S-Me (III)
worin R die oben angegebene Bedeutung hat und Me für ein Alkalimetallkation steht, umsetzt, und die auf diese Weise erhaltene Verbindung der Formel IV 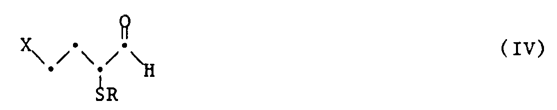 worin X und R die oben angegebene Bedeutung haben, in einem inerten Lösungsmittel in Gegenwart einer Base in eine Verbindung der Formel I überführt.

2. Verfahren nach Anspruch 1, dadurch gekennzeichnet, dass man eine Verbindung der Formel II, worin X und Y die im Anspruch 1 angegebene Bedeutung haben, mit einer Verbindung der Formel III, worin R und Me die im Anspruch 1 angegebene Bedeutung haben, ohne Isolierung der Zwischenprodukte der Formel IV in Gegenwart einer Base in einem inerten Lösungsmittel zu einer Verbindung der Formel I umsetzt.

3. Verfahren nach Anspruch 1, dadurch gekennzeichnet, dass man es bei einer Temperatur von -10°C bis +100°C durchführt.

4. Verfahren nach Anspruch 1, dadurch gekennzeichnet, dass man es bei einer Temperatur von -10°C bis +50°C durchführt.

5. Verfahren nach Anspruch 1, dadurch gekennzeichnet, dass man als inertes Lösungsmittel einen Alkohol mit 1 bis 4 Kohlenstoffatomen, Wasser, Tetrahydrofuran oder Dioxan verwendet.

6. Verfahren nach Anspruch 1, dadurch gekennzeichnet, dass man als Lösungsmittel Methanol, Tetrahydrofuran oder Wasser verwendet.

7. Verfahren nach Anspruch 1, dadurch gekennzeichnet, dass man als Basen ein Oxid, Hydroxid, Carbonsäuresalz, Carbonat oder Alkoholat eines Alkali- oder Erdalkalimetalls verwendet.

8. Verfahren nach Anspruch 1, dadurch gekennzeichnet, dass man als Base Natriummethylat, Natriumhydroxid oder Kaliumhydroxid verwendet.

9. Verfahren nach Anspruch 1, dadurch gekennzeichnet, dass R für C₁-C₄-Alkyl steht.

10. Verfahren nach Anspruch 1, dadurch gekennzeichnet, dass Me für Natrium oder Kalium steht.

11. Verfahren nach Anspruch 1, dadurch gekennzeichnet, dass X für Chlor und Y für Chlor oder Brom stehen.

12. Verfahren nach Anspruch 1, dadurch gekennzeichnet, dass X und Y für Chlor stehen.

13. Verbindungen der Formel IV 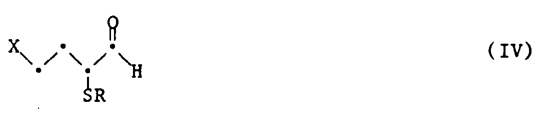 worin R und X die unter Anspruch 1 angegebene Bedeutung haben, als Zwischenprodukte.

14. Verbindungen der Formel IV nach Anspruch 13, dadurch gekennzeichnet, dass R für C₁-C₄-Alkyl und X für Cl steht.

15. Verbindungen der Formel II 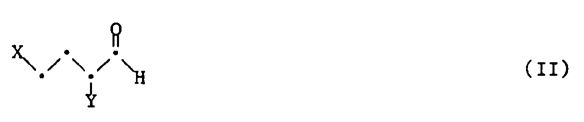 worin X und Y die im Anspruch 1 angegebene Bedeutung haben.

16. Verbindungen der Formel II gemäss Anspruch 15, dadurch gekennzeichnet, dass X für Chlor und Y für Chlor oder Brom stehen.

17. Verbindungen der Formel II gemäss Anspruch 15, dadurch gekennzeichnet, dass X und Y für Chlor stehen.

18. Verfahren nach Anspruch 2, dadurch gekennzeichnet, dass man es bei einer Temperatur von -10°C bis +50°C in Gegenwart von Natriumhydroxid oder Kaliumhydroxid in Wasser oder Methanol durchführt.

19. Verfahren nach Anspruch 18, dadurch gekennzeichnet, dass Me für Natrium, X für Chlor, Y für Chlor oder Brom und R für C₁-C₄-Alkyl steht.

## Claims

1. A process for the preparation of a compound of formula I 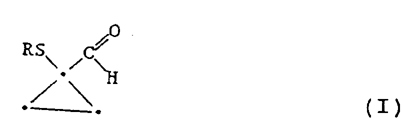 wherein R is C₁-C₄alkyl or benzyl, which comprises reacting a butanal of formula II 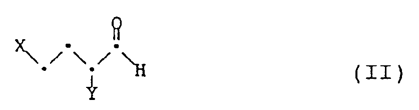 wherein X and Y are each independently of the other chlorine, bromine or iodine, in an inert solvent, with a compound of formula III
R-S-Me (III)
wherein R is as defined above and Me is an alkali metal cation, and converting the resultant compound of formula IV 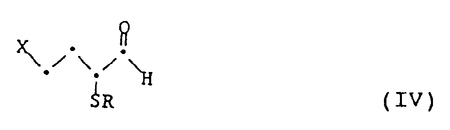 wherein X and R are as defined above, in an inert solvent and in the presence of a base into a compound of formula I.

2. A process according to claim 1, which comprises reacting a compound of formula II, wherein X and Y are as defined in claim 1, with a compound of formula III, wherein R and Me are as defined in claim 1, without isolation of the intermediate of formula IV, in an inert solvent and in the presence of a base, to give a compound of formula I.

3. A process according to claim 1 which is carried out in the temperature range from -10°C to +100°C.

4. A process according to claim 1 which is carried out in the temperature range from -10°C to +50°C.

5. A process according to claim 1, wherein the inert solvent used is a C₁-C₄alcohol, water, tetrahydrofuran or dioxane.

6. A process according to claim 1, wherein the solvent used is methanol, tetrahydrofuran or water.

7. A process according to claim 1, wherein the base used is an oxide, hydroxide, carboxylate, carbonate or alcoholate of an alkali metal or alkaline earth metal.

8. A process according to claim 1, wherein the base used is sodium methylate, sodium hydroxide or potassium hydroxide.

9. A process according to claim 1, wherein R is C₁-C₄alkyl.

10. A process according to claim 1, wherein Me is sodium or potassium.

11. A process according to claim 1, wherein X is chlorine and Y is chlorine or bromine.

12. A process according to claim 1, wherein X and Y are chlorine.

13. A compound of formula IV 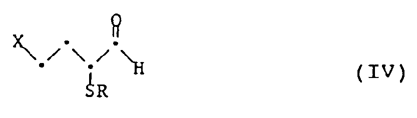 wherein R and X are as defined in claim 1, as intermediate.

14. A compound of formula IV according to claim 13, wherein R is C₁-C₄alkyl and X is chlorine.

15. A compound of formula II 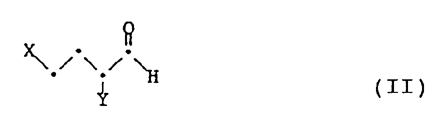 wherein X and Y are as defined in claim 1.

16. A compound of formula II according to claim 15, wherein X is chlorine and Y is chlorine or bromine.

17. A compound of formula II according to claim 15, wherein X and Y are chlorine.

18. A process according to claim 2 which is carried out in the temperature range from -10°C to +50°C, in the presence of sodium hydroxide or potassium hydroxide, in water or methanol.

19. A process according to claim 18, wherein Me is sodium, X is chlorine, Y is chlorine or bromine, and R is C₁-C₄alkyl.

## Revendications

1. Procédé de préparation des composés de formule I 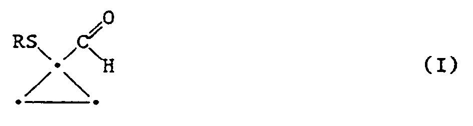 dans laquelle
R représente un groupe alkyle en C 1-C 4 ou benzyle, caractérisé en ce que l'on fait réagir un butanal de formule II 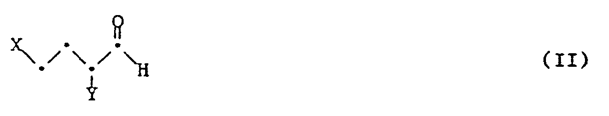 dans laquelle X et Y représentent chacun, indépendamment l'un de l'autre, le chlore, le brome ou l'iode, dans un solvant inerte, avec un composé de formule III
R-S-Me (III)
dans laquelle R a les significations indiquées ci-dessus et Me représente un cation de métal alcalin, ce qui donne le composé de formule IV 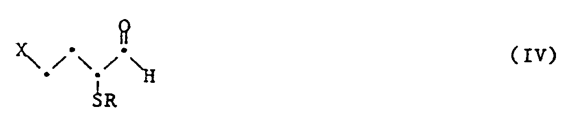 dans laquelle X et R ont les significations indiquées ci-dessus, qu'on convertit en un composé de formule I dans un solvant inerte en présence d'une base.

2. Procédé selon la revendication 1, caractérisé en ce que l'on fait réagir un composé de formule II dans laquelle X et Y ont les significations indiquées dans la revendication 1 avec un composé de formule III dans laquelle R et Me ont les significations indiquées dans la revendication 1 sans isoler les produits intermédiaires de formule IV, en présence d'une base et dans un solvant inerte, la réaction donnant un composé de formule I.

3. Procédé selon la revendication 1, caractérisé en ce que l'on opère à une température de -10 à +100°C.

4. Procédé selon la revendication 1, caractérisé en ce que l'on opère à une température de -10 à +50°C.

5. Procédé selon la revendication 1, caractérisé en ce que l'on utilise en tant que solvant inerte un alcool en C 1-C 4, l'eau, le tétrahydrofuranne ou le dioxanne.

6. Procédé selon la revendication 1, caractérisé en ce que l'on utilise en tant que solvant le méthanol, le tétrahydrofuranne ou l'eau.

7. Procédé selon la revendication 1, caractérisé en ce que l'on utilise en tant que base un oxyde,un hydroxyde, un carboxylate, un carbonate ou un alcoolate d'un métal alcalin ou alcalino-terreux.

8. Procédé selon la revendication 1, caractérisé en ce que l'on utilise en tant que base le méthylate de sodium, l'hydroxyde de sodium ou l'hydroxyde de potassium.

9. Procédé selon la revendication 1, caractérisé en ce que R représente un groupe alkyle en C 1-C 4.

10. Procédé selon la revendication 1, caractérisé en ce que Me représente le sodium ou le potassium.

11. Procédé selon la revendication 1, caractérisé en ce que X représente le chlore et Y le chlore ou le brome.

12. Procédé selon la revendication 1, caractérisé en ce que X et Y représentent tous deux le chlore.

13. Composés de formule IV 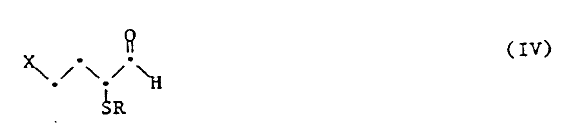 dans laquelle R et X ont les significations indiquées dans la revendication 1, en tant que produits intermédiaires.

14. Composés de formule IV de la revendication 13, caractérisés en ce que R représente un groupe alkyle en C 1-C 4 et X le chlore.

15. Composés de formule II 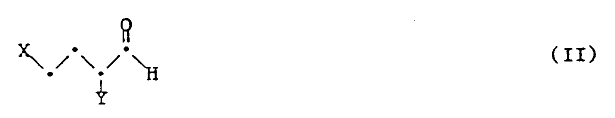 dans laquelle X et Y ont les significations indiquées dans la revendication 1.

16. Composés de formule II de la revendication 15, caractérisés en ce que X représente le chlore et Y le chlore ou le brome.

17. Composés de formule II de la revendication 15, caractérisés en ce que X et Y représentent tous deux le chlore.

18. Procédé selon la revendication 2, caractérisé en ce que l'on opère à une température allant de -10 à +50°C en présence d'hydroxyde de sodium ou d'hydroxyde de potassium dans l'eau ou dans le méthanol.

19. Procédé selon la revendication 18, caractérisé en ce que Me représente le sodium, X le chlore, Y le chlore ou- le brome et R un groupe alkyle en C 1-C 4.
